# EUROPEAN PATENT SPECIFICATION

(11) **EP 1 799 111 B1**
(45) Date of publication and mention of the grant of the patent: **02.09.2015**
(21) Application number: 05787200.4
(22) Date of filing: 03.10.2005
(51) Int. Cl.: A61B 8/00, G01S 7/52, G06F 19/00

(54) **ULTRASONIC DIAGNOSTIC SYSTEM WITH FLEXIBLE EXAMINATION PROTOCOLS AND REPORT GENERATION**
ULTRASCHALLDIAGNOSESYSTEM MIT FLEXIBLEN UNTERSUCHUNGSPROTOKOLLEN UND BERICHTERZEUGUNG
SYSTEME DE DIAGNOSTIC ULTRASONORE COMPORTANT DES PROTOCOLES D'EXAMEN FLEXIBLES ET PRODUISANT DES RAPPORTS

(30) Priority: 08.10.2004 US 617491 P
(43) Date of publication of application: 27.06.2007
(73) Proprietor: Koninklijke Philips N.V., 5656 AE Eindhoven (NL)
(72) Inventor: SAAD, Ashraf, Bothell, Washington 98041-3003 (US); SKYBA, Dan, Bothell, Washington 98041-3003 (US)
(86) International application number: PCT/IB2005/053250
(87) International publication number: WO 2006/038181

(56) References cited:
- US-A- 6 149 594
- US-A1- 2003 026 464
- US-B1- 6 306 089
- US-B1- 6 458 081

## Description

This invention relates to ultrasonic diagnostic imaging systems and, in particular, to ultrasonic imaging systems which conduct examinations in accordance with specified clinical protocols.

In the past, ultrasound machines have been used to image any anatomy that could be clearly seen with the probe set available for the particular machine. But as ultrasonic diagnosis has become for sophisticated and the technology more refined, ultrasound machines became configured for certain types of examinations such as obstetrics, cardiology, vascular and radiology. In the recent past the practice of ultrasound diagnosis has become more standardized, with specifically designed image acquisition protocols for patients with specific symptoms. For example, a general abdominal exam protocol may call for the acquisition of specified views of the liver, kidneys, gall bladder and pancreas. A general vascular exam may call for the acquisition of specified views of the carotid artery and vasculature of the limbs of the body. Ultrasound machine manufacturers have followed this trend by providing their machines with pre-programmed exam protocols which guide the sonographer through these specified imaging sequences and produce report automatically tailored to the specified information. Such pre-programmed protocols have improved the efficiency of ultrasound exams.

While improving the efficiency of an examination, pre-programmed protocols, particularly those for a general survey exam, are generally designed to step the sonographer through a series of views, measurements, and calculations in regions of the body to determine whether the imaged anatomy is normal or exhibits suspect characteristics. At the end of the protocol the sonographer is expected to review the findings of the exam and, if a suspicious condition is indicated, go back to the suspect anatomy and conduct more detailed imaging and analysis. However, the sonographer may desire to examine suspect anatomy in more detail at the time a possible problem is indicated, particularly if a marginal condition is indicated. This will generally require that the exam protocol be aborted, and the guided examination of other anatomy by the protocol terminated. It would be desirable to be able to modify the protocol during its execution to enable the sonographer to branch into selective, more detailed exam steps and then to continue with the exam protocol to its conclusion. It would further be desirable for the results of the spontaneous detailed exam steps to be recorded in any automated report generated as a result of the protocol execution.

In accordance with the principles of the present invention an ultrasonic diagnostic imaging system is provided with an examination protocol which is variable during the execution of the protocol. The pre-programmed protocol can be augmented by additional examination protocol steps which are user-definable. The added protocol steps are context-based so as to automatically guide the sonographer through additional detailed analysis of the anatomy for which they are employed. The results of the added protocol steps flow directly into a system-generated report in the proper sequence of the protocol exam results.

Relevant prior art is disclosed by US6458081B1 and US2003/026464A1.

The invention is defined by claim 1.

In the drawings:
FIGURE 1 shows an ultrasonic diagnostic imaging system constructed in accordance with the principles of the present invention.
FIGURE 2 illustrates in block diagram form the functional elements of the ultrasound system of FIGURE 1 and ancillary peripheral devices useful in an implementation of the present invention.
FIGURE 3 is a schematic illustration of the carotid artery.
FIGURE 4 illustrates a touchscreen containing vascular exam protocol buttons in accordance with one embodiment of the present invention.
FIGURE 5 illustrates the taking of two velocity measurements of the common carotid artery during a vascular exam.
FIGURE 6 illustrates a touchscreen containing vascular exam protocol buttons with new buttons for additional protocol steps added by the sonographer.
FIGURE 7 illustrates a pop-up display for a sonographer to label a user-created measurement protocol step.
FIGURE 8 is a schematic illustration of a stenotic carotid artery.
FIGURE 9 illustrates a vascular exam report including the results of a measurement protocol step added by the sonographer during the course of the execution of the protocol.
FIGURE 10 is a schematic illustration of a stenotic vessel which is to be analyzed.
FIGURE 11 illustrates a touchscreen containing user-created protocol steps for a % diameter reduction measurement of a stenotic vessel.
FIGURE 12 illustrates an ultrasound image display containing the distance caliper tool invoked by the user-created % diameter reduction protocol step.
FIGURE 13 illustrates a touchscreen containing user-created protocol steps for a % area reduction measurement of a stenotic vessel.
FIGURE 14 illustrates an ultrasound image display containing the ellipse and trace tools invoked by the user-created % area reduction protocol step.
FIGURE 15 illustrates a vascular exam report containing the results of the user-created % diameter and % area reduction steps.

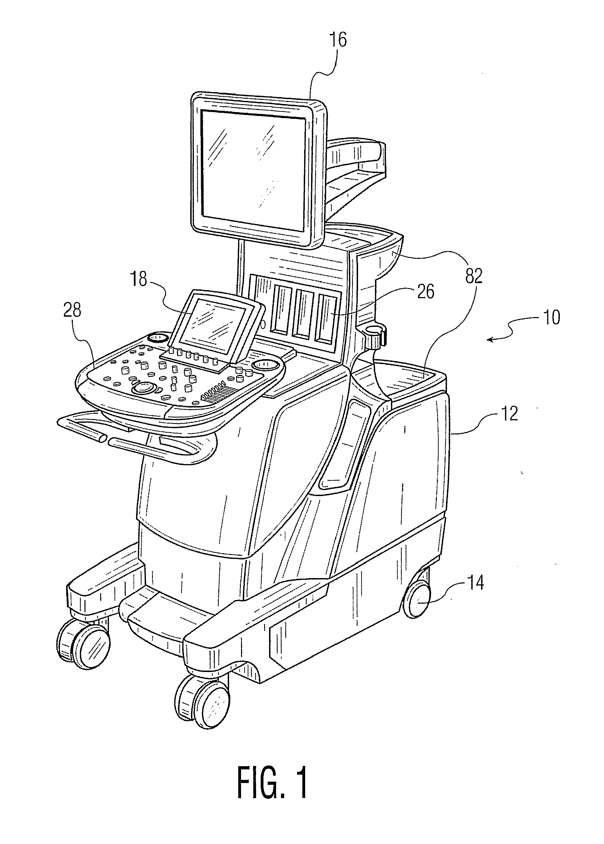

An ultrasound imaging system 10 constructed in accordance with one embodiment of the invention is illustrated FIGURE 1. The system 10 includes a chassis 12 containing most of the electronic circuitry for the system 10. The chassis 12 is mounted on a cart 14, and a display 16 is mounted on the chassis 12. Different imaging probes may be plugged into three connectors 26 on the chassis. The chassis 12 includes a keyboard and controls, generally indicated by reference numeral 28, for allowing a sonographer to operate the ultrasound system 10 and enter information about the patient or the type of examination that is being conducted. At the back of the control panel 28 is a touchscreen display 18 on which programmable softkeys are displayed for protocol execution in accordance with the present invention. The sonographer selects a softkey on the touchscreen display 18 simply by touching the image of the softkey on the display.

In operation, a probe is placed against the skin of a patient (not shown) and either held stationery or moved to acquire an image of blood or tissues beneath the skin. The image is presented on the display 16, and it may be recorded by a recorder (not shown) placed on one of the accessory shelves 82. The system 10 may also record or print a report containing text and images. Data corresponding to the image may also be downloaded through a suitable data link, such as the Internet or a local area network. The type of image shown on the display 16, the type of report recorded or printed, and the type of data downloaded will depend on the type of ultrasound examination that is being conducted.

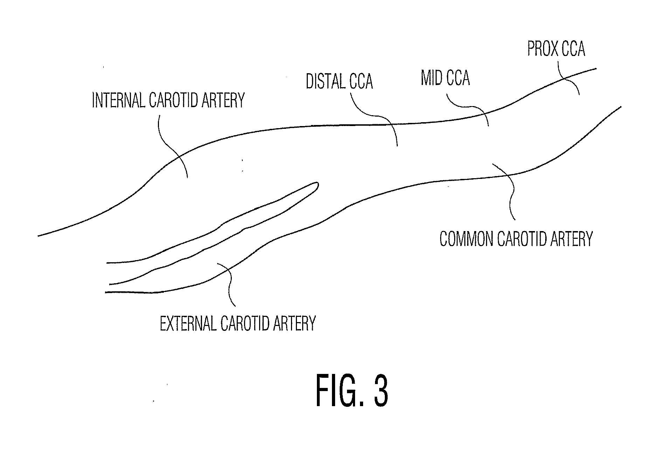

The above-described components of the imaging system 10 are conventional and are commonly used to obtain ultrasound images. The imaging system 10 according to one embodiment of the invention uses examination protocols, which may be standardized throughout the healthcare field, to automatically guide the sonographer through standard ultrasound exams. The protocols are used in a manner that will be explained in detail in connection with FIGURES 3-15.

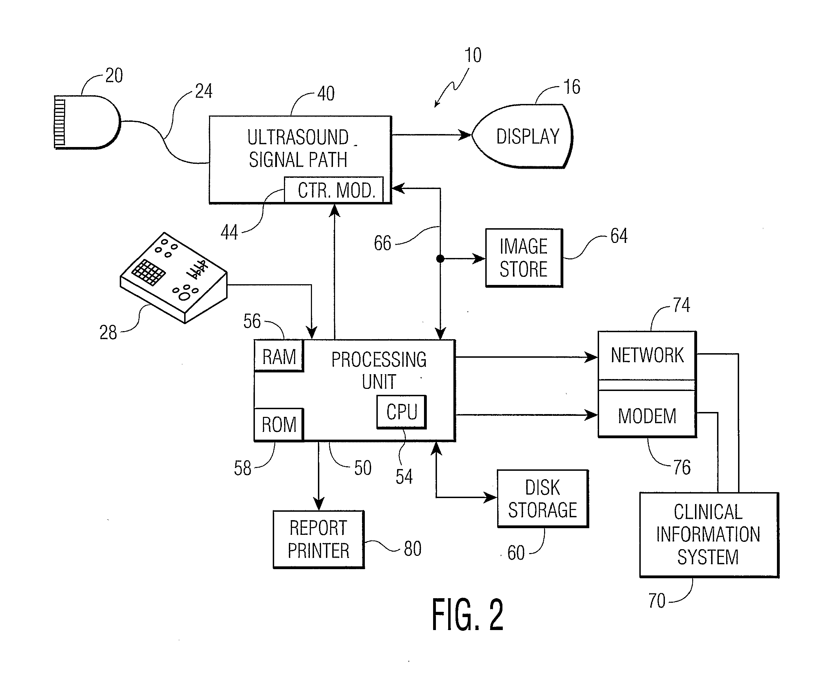

The elements of the ultrasound imaging system 10 are illustrated in greater detail in FIGURE 2. An ultrasound imaging probe 20 is coupled by a cable 24 to one of the connectors 26 which connect to an ultrasound signal path 40 of conventional design. Although one ultrasound imaging probe is shown in FIGURE 2, it will be understood that other types of imaging probes can and generally will be used depending upon the type of ultrasound examination being conducted. In the embodiment shown in FIGURE 2, the imaging probe 20 and all other imaging probes that will be used with the system 10 preferably provide probe identifying signals to a processing unit 50 to allow the processing unit 50 to determine the type of probe 20 currently being used.

As is well-known in the art, the ultrasound signal path 40 includes a transmitter (not shown) coupling electrical signals to the probe 20, an acquisition unit (not shown) that receives electrical signals from the probe 20 corresponding to ultrasound echoes, a signal processing unit (not shown) that processes the signals from the acquisition unit to perform a variety of functions such as isolating returns from specific depths or isolating returns from blood flowing through vessels, and a scan converter (not shown) that converts the signals from the signal processing unit so that they are suitable for use by the display 16. The processing unit in this embodiments is capable of processing both B mode (structural) and Doppler signals for the production of various B mode and Doppler displays including spectral Doppler. The ultrasound signal path 40 also includes a control module 44 that interfaces with the processing unit 50 to control the operation of the above-described units. The ultrasound signal path 40 may, of course, contain components in addition to those described above, and, it suitable instances, some of the components described above may be omitted.

The processing unit 50 contains a number of components, including a central processor unit ("CPU") 54, random access memory ("RAM") 56, and read only memory ("ROM") 58, to name a few. As is well-known in the art, the ROM 58 stores a program of instructions that are executed by the CPU 54, as well as initialization data for use by the CPU 54. The RAM 56 provides temporary storage of data and instructions for use by the CPU 54. The processing unit 50 interfaces with a mass storage device such as a disk drive 60 for permanent storage of data, such as data corresponding to ultrasound images obtained by the system 10. However, such image data is initially stored in an image storage device 64 that is coupled to a signal path 66 extending between the ultrasound signal path 40 and the processing unit 50. The storage drive 60 also preferably stores protocols which may be called up and initiated to guide the sonographer through various ultrasound exams. However, in another embodiment the protocols are stored in a clinical information system 70 that may be accessed through suitable means such as a local area network 74, a modem 76 or a wireless communication link (not shown). The central storage method enables a facility like a hospital to store all the standardized protocols for all of the diagnostic systems in the facility, to control their modification uniformly, and to rapidly disburse the protocols to the ultrasound systems and users in the facility. Once a protocol has been retrieved by the system, it can be executed under control of the processing unit 50 to carry out the diagnostic exam of the protocol.

The processing unit 50 also interfaces with the keyboard and controls 28, which may be used to execute the protocols. The keyboard and controls 28 may also be manipulated by the sonographer to cause the ultrasound system to produce automatically generated reports at the conclusion of an examination. The processing unit 50 preferably interfaces with a report printer 80 that prints reports containing text and one or more images. The type of reports provided by the printer 80 depends on the type of ultrasound examination that was conducted by the execution of a specific protocol.

One example of the execution of a modifiable protocol in accordance with the principles of the present invention is illustrated with reference to FIGURES 3-9. FIGURE 3 is an illustration of the carotid artery. A standard peripheral vascular examination protocol might call for measurements to be taken in the three branches of the carotid artery shown in this illustration: The internal carotid artery, the external carotid artery, and the common carotid artery. In the common carotid artery (CCA) three measurements are commonly taken: one at the distal CCA, one at the mid CCA, and one at the proximal CCA.

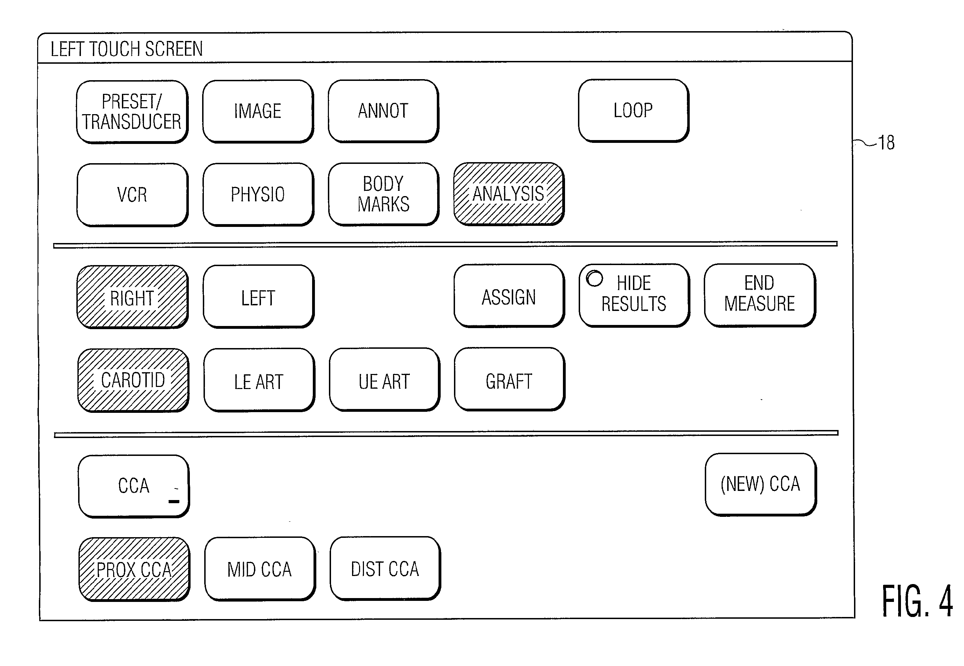

FIGURE 4 shows the touchscreen display 18 for a peripheral vascular examination protocol in a constructed embodiment of the present invention. The constructed embodiment has two such touchscreen displays and the protocol is shown on the left touchscreen on the ultrasound system. The touchscreen display 18 is divided into three areas, each having two rows of softkeys. The upper area has high level key for selecting controls for different functions such as imaging (the "Image" button) and VCR control (the "VCR" button). Protocols are selected by touching the "Analysis" button, which is shown in a dark color when selected as it is in this illustration. In this example there are four types of exam protocols which may be initiated through the Analysis button, a carotid artery exam, a lower extremity arterial exam ("LE Art" button), an upper extremity arterial exam ("UE Art" button), and a grafts exam, which are shown in the central area of the display. Other choices which may be presented to the sonographer could be examinations of the venous structures of the body. In this example the Carotid button has been selected to initiate a carotid exam protocol and the protocol begins with the right carotid artery as shown by the darkened "Right" button. After the right carotid artery has been examined and measurements taken, the "Left" button will be touched to continue the protocol on the left carotid artery.

The first area of the carotid artery to be examined in this protocol example is the common carotid artery as indicated by the "CCA_ button in the lower area of the touchscreen display. As indicated in FIGURE 3, measurements will be taken at three points in the CCA which are selected by the three buttons at the bottom of the display. In this example the proximal CCA measurements will be taken first as indicated by the darkened "Prox CCA" button. To the right of the standard CCA_ measurement button is a "(New) CCA" template button by which the standard measurements can be augmented as described below.

It may be seen that the touchscreen buttons on the touchscreen display 18 are arranged hierarchically. The Analysis button for the selection of protocols is at the top, the button for the Carotid exam and the button for the Right carotid are in the center of the display, and the button for the CCA measurements of the right carotid and the three points where CCA measurements are to be taken are at the bottom. It is apparent that a tree and branches display structure could alternatively be used for this hierarchical display similar to those used for the directory and file structure display on a computer. The embodiment of FIGURE 4 combines attributes of a hierarchically structure in a functional touchscreen display.

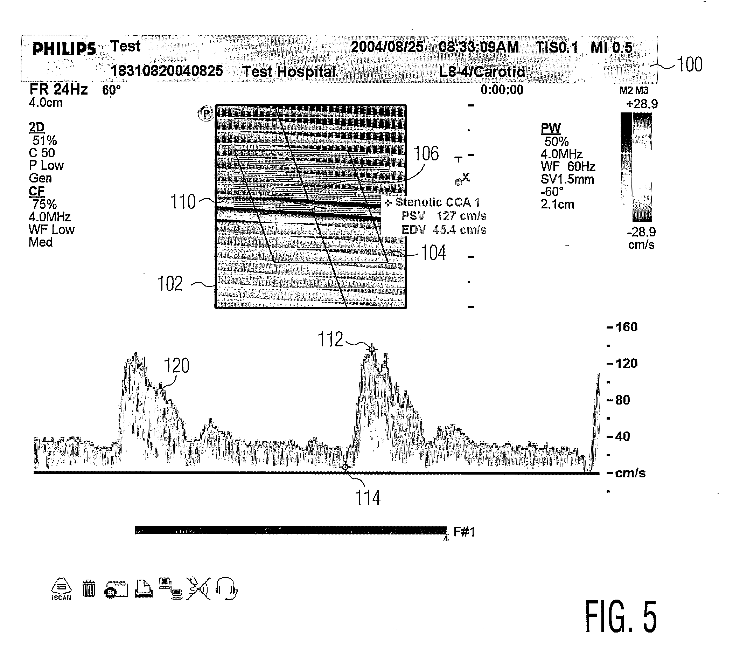

When the sonographer wants to take measurements of the CCA, an ultrasound image of the CCA is acquired as illustrated in FIGURE 5. In FIGURE 5 a Doppler box 104 is shown over the ultrasound image 102 of the carotid artery 110. (For clarity of illustration a schematic of an ultrasound image is shown in the drawings.) Also shown is a sample volume cursor 106 which the sonographer can align with the point in the CCA where the proximal measurement is to be taken. Below the ultrasound image 102 is a spectral display 120 acquired at the point indicated by the sample volume cursor 106. After the sonographer aligns the cursor 106 at the desired sample point for the measurement and an acceptable spectral display is acquired, the sonographer freezes the display and takes the proximal CCA measurements by touching the Prox CCA button on the touchscreen display. The sonographer does this by marking the peak systolic velocity (PSV) and the end diastolic velocity (EDV) on the spectral display as shown at 112 and 114. As FIGURE 5 shows, the quantified measurement values of the PSV and the EDV appear next to the ultrasound image 102 for review by the sonographer. If the sonographer is satisfied with these measurements the sonographer touches the "End Measure" button on the touchscreen display, the proximal CCA measurements are saved by the system, and a small checkmark (not shown) appears in the lower right-hand corner of the Prox CCA button, indicating that this measurement has been completed. The sonographer may then touch the Mid CCA or Dist CCA button to take another CCA measurement.

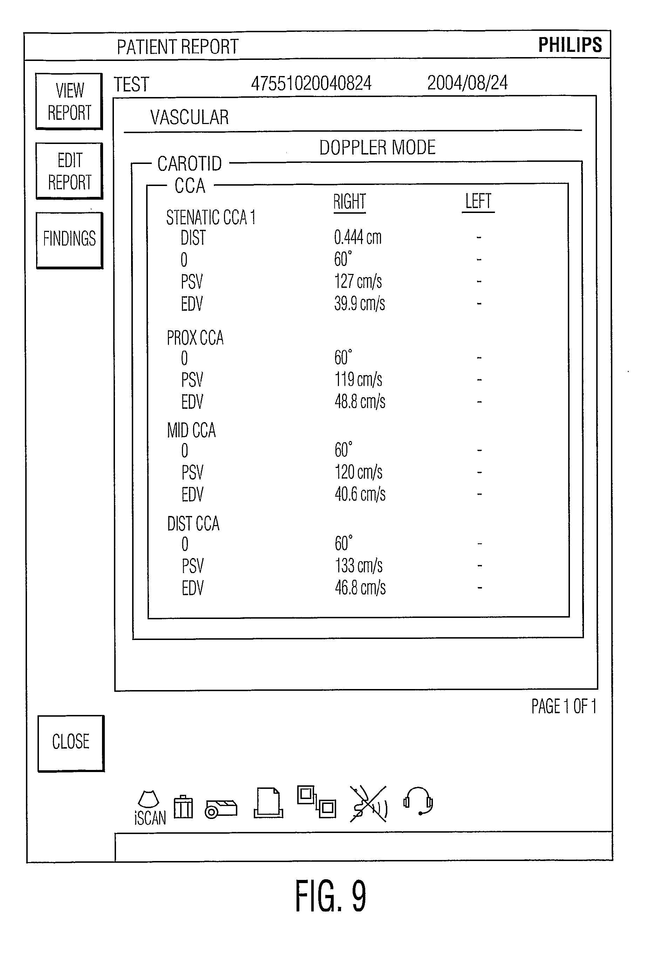

The protocol continues in this manner until all the protocol measurements for the right carotid artery have been taken. The sonographer then touches the Left button to take the measurements of the left carotid artery. As the measurements are taken they are stored by the system in a report database on the ultrasound system. When the sonographer has completed the exam the sonographer can go to the Report Menu on the system and view, save, and print an automatically generated report of the examination. A typical automatically generated report of a vascular exam is shown in FIGURE 9, which shows measurements taken at the proximal, mid, and distal CCA points of the right carotid artery.

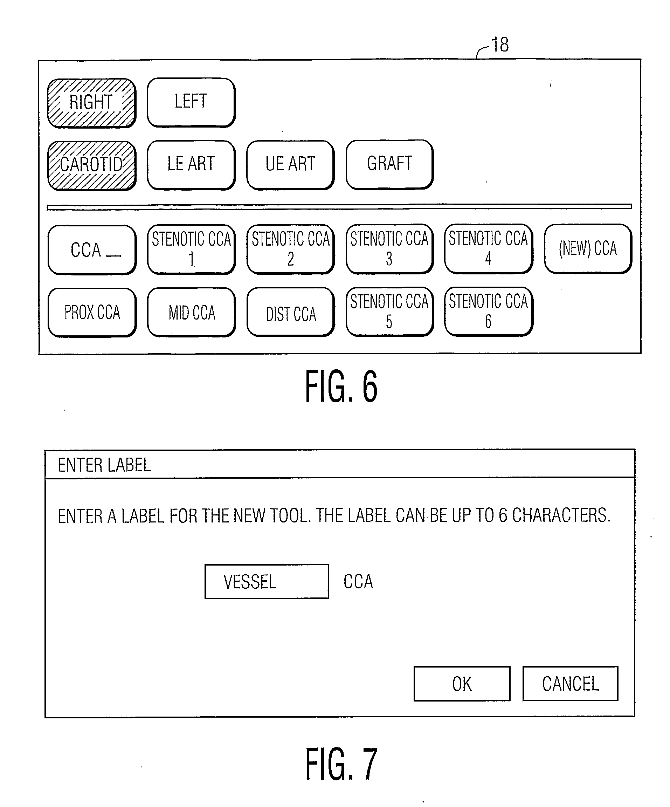

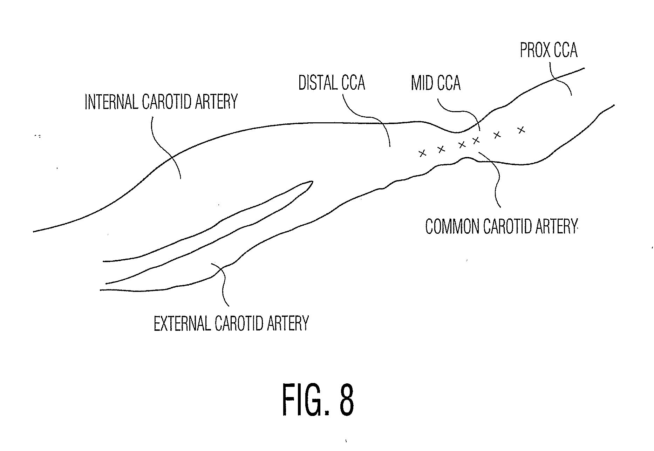

However, suppose that the measurements taken of the CCA indicate a stenosis in the mid region of the artery. In such case the sonographer will want to examine that region of the artery in greater detail. FIGURE 8, for instance, illustrates a stenosis at the CCA and indicates six points in the mid CCA where additional measurements should be taken to better define the flow profile at the stenosis. In prior art system the sonographer would have to either complete the carotid protocol and then begin a more detailed analysis in the mid CCA. Alternatively, the sonographer could abort the protocol, take the more detailed measurements, and then begin the protocol again. In accordance with the present invention, the sonographer can modify the pre-programmed carotid protocol by touching the "(New) CCA" template button, which creates a new measurement step in the protocol. The new measurement step is context-driven, and hence the system is displaying a (New) CCA button when CCA measurements are being taken. When the sonographer touches the (New) CCA template button a box appears on a system display as shown in FIGURE 7, asking the sonographer to define the new measurement step with a label. The placeholder label "Vessel" is shown in the box where the sonographer is to type a desired name for the new measurement. In this example the sonographer types in "Stenotic CCA 1", at which moment a newly defined button appears on the touchscreen 18 as illustrated by the "Stenotic CCA 1" button above the Mid CCA button in FIGURE 9. The sonographer then acquires a Doppler image of the CCA, positions the sample volume cursor, and freezes the image as discussed above. When the sonographer touches the newly defined button, the system launches the measurement tools necessary for a CCA measurement, which in this example include velocity measurements and a caliper tool. Touching the new CCA button automatically invokes these operations without the involvement of the sonographer, reducing the time needed to conduct the exam. In this example the sonographer takes the measurements for Stenotic CCA 1, the new button is checked to indicate that the measurements have been taken, and the sonographer proceeds to the next measurement. FIGURE 8 shows that six such measurement points are to be analyzed, and the ultrasound system enables this to be done by allowing the sonographer to create six new protocol step buttons from the (New) CCA template button as shown by Stenotic CCA buttons 1-6 in FIGURE 6.

FIGURE 9 shows that, when the ultrasound system has an automatic report generation function linked to the customized protocol feature, the newly created protocol step is automatically included in the CCA measurements section of the report as seen by the Stenotic CCA 1 measurements at the top of the report screen. In accordance with a further aspect of the present invention, the sonographer can also augment the standard measurement tools of a protocol step.
In this example the sonographer has touched the "Assign" button while taking the Stenotic CCA 1 measurements, and has added a distance measurement to this protocol step. This addition will launch a distance measurement tool by which the sonographer can measure the distance across the CCA at the point of the measurement. As FIGURE 9 shows, the distance measurement taken at the point of the Stenotic CCA 1 measurement is 0.444 cm. Thus, the sonographer can add new steps to the standard exam protocol and can also incorporate selected measurements into newly added protocol steps so as to better diagnose the patient's condition.

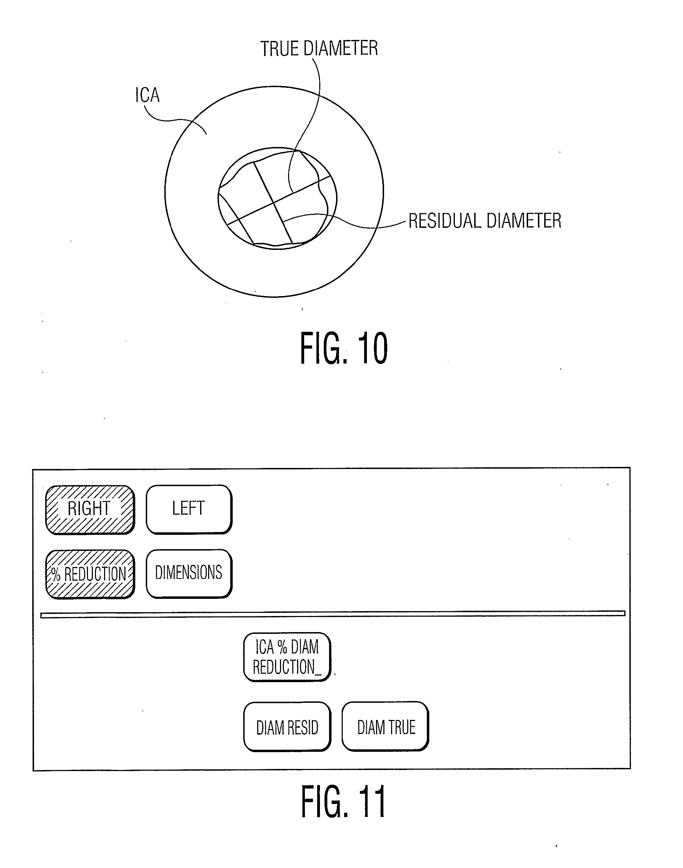

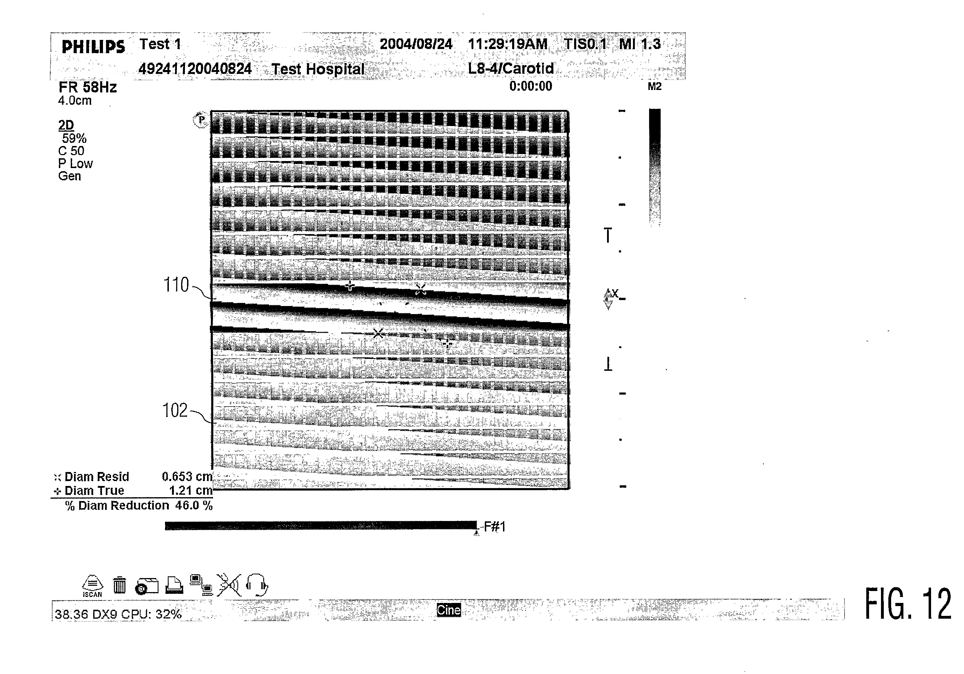

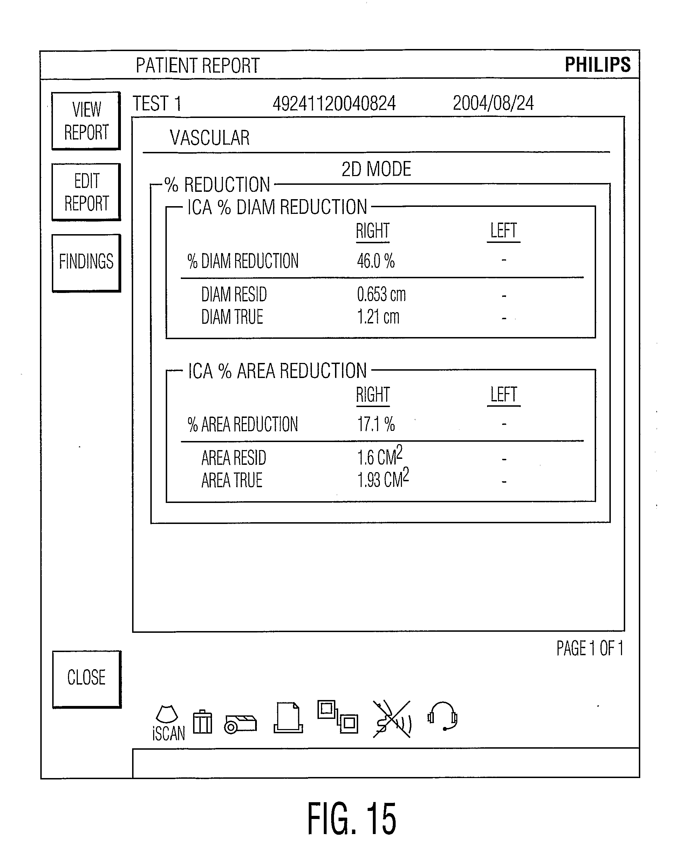

As another example suppose that a sonographer has detected reduced blood flow in the internal carotid artery (ICA; see FIGURE 3) wants to assess the percent reduction of the right carotid artery ICA caused by plaque build-up in the ICA. In this case the sonographer touches the Right button to display optional button templates for carotid artery calculations, one of which is the "% Reduction" button shown in FIGURE 11. The % Reduction button has two template buttons, one for diameter reduction and one for area reduction. The % diameter reduction template guides the sonographer in measuring the true diameter and the residual diameter of the vessel, then computes the percent reduction in effective vessel diameter as a function of the two measurements. This is shown in the illustration of an ICA in cross-section. The true diameter is the actual diameter of the vessel in the absence of any plaque. The residual diameter is the diameter of the vessel lumen remaining after the plaque is present. When the sonographer touches the (New) % diameter reduction template button a box appears (see FIGURE 7) to request that the sonographer type in a name for the new diameter reduction template step. In this example the sonographer types "ICA % Diam Reduction," which creates a new protocol step button as shown in FIGURE 11. When the sonographer touches the new ICA % Diam Reduction button, two associated measurement buttons appear, the Diam Resid button and the Diam True button. When the sonographer touches the Diam Resid button a distance caliper tool is launched with the ultrasound image 102 as shown in FIGURE 12. This tool enables a distance measurement to be made of the residual vessel diameter on the image of the carotid artery 110, which is marked with an "X" sign as shown in the center of FIGURE 12. The caliper tool enables the sonographer to place these X markers on the appropriate points of the anatomy in the image, and the quantified numerical results of the residual diameter measurement appears to the left of the ultrasound image 102. When the sonographer touches the Diam True button another distance caliper tool is launched for the true diameter measurement, this one marked with a "+" sign. The sonographer aligns the + signs with the proper points on the ultrasound image and touches the ICA % Diam Reduction button. The ultrasound system then uses the two diameter measurements just taken and computes the % diameter reduction of the vessel, which appears just below the two measurements as shown in FIGURE 12. When the sonographer touches the End Measure button the two measurements and the % diameter reduction calculation are saved by the system and a check mark appears on the ICA % Diam Reduction button to indicate that this protocol step has been completed. The measurement and calculation results will now appear in the linked automatically generated report as shown in FIGURE 15.

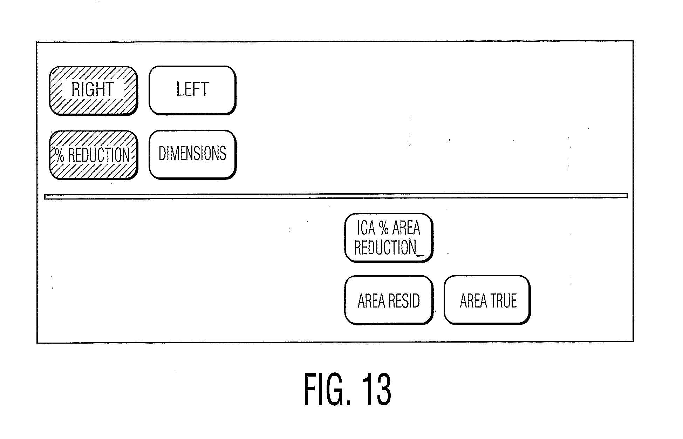

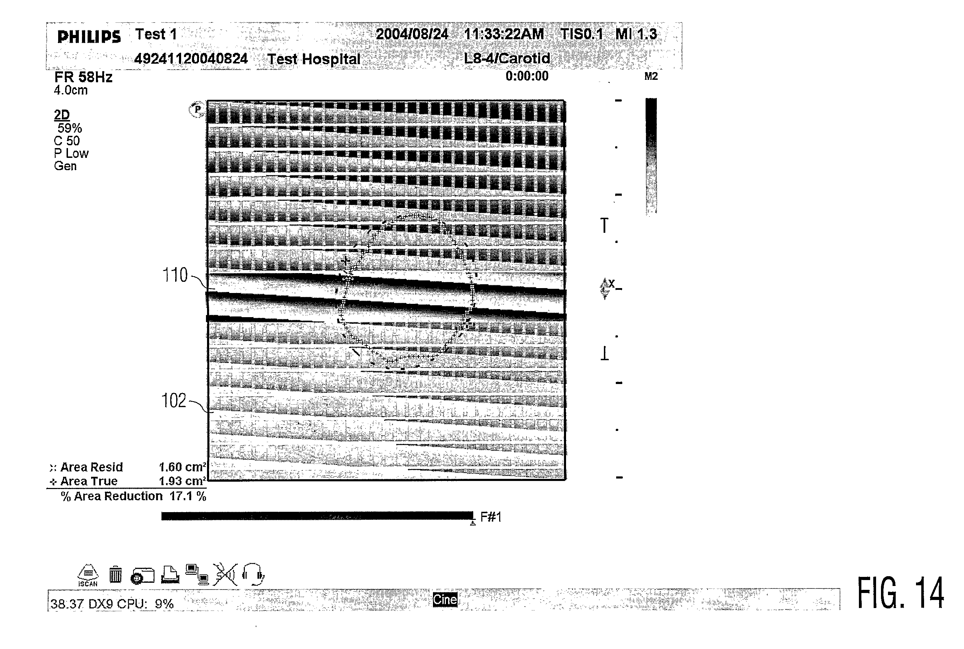

As an illustration of a further embodiment of the present invention, suppose that the sonographer wants to assess the % area reduction of the vessel. The sonographer will touch the (New) % Area Reduction template button, which will display a box in which the sonographer can name the new area reduction step (see FIGURE 7). In this example the sonographer types in "ICA % Area Reduction" and the named new protocol step button appear as shown in FIGURE 13. When the sonographer touches the new ICA % Area Reduction button, two associated measurement buttons appear, the Area Resid button and the Area True button. Touching the Area True button launches an ellipse tool, since the ultrasound system knows that the true area of a vessel is measured with an ellipse graphic. The ellipse graphic is produced over the carotid artery 110 in the ultrasound image 102 as shown in FIGURE 14 by the dashed lines with the + signs. The sonographer grabs the ellipse with a cursor and fits it to the actual inner circumference of the blood vessel. The quantified area measurement obtained by the fitted ellipse appears numerically to the left of the ultrasound image 102. The sonographer touches the Diam Resid button which launches a tracing tool over the ultrasound image. The sonographer then traces the actual inner diameter of the stenosis with the tool, as indicated by the dotted line with the X on it in FIGURE 14. The Area Resid measurement appears numerically with the Area True measurement, and the calculated % Area Reduction calculation appears below the two measurements. If the sonographer is satisfied with the results the sonographer touches the End Measure button (see FIGURE 4) to save the results of the protocol step for the automatically generated report and a check mark appears on the ICA % Area Reduction button, marking the step as complete. A sample page of an automatically generated report with the results of these two protocol steps shown on the page is illustrated in FIGURE 15.

At the conclusion of the ultrasound exam the modified exam protocol can be deleted from the ultrasound system so that the selection of the exam protocol for another exam will initiate the same standardized exam protocol which was initiated at the start of the modified exam. Alternatively, the sonographer may decide to save the modified exam protocol, which may be used in the future, for example, to examine the same patient at a later point in time in accordance with the same modified exam protocol.

## Claims

1. An ultrasonic diagnostic imaging system (10) adapted to execute a pre-programmed protocol to guide a user through the steps of an ultrasound examination comprising:
a display (16) of the steps of an exam protocol;
an optional user selection by which a user can add a new step to the exam protocol during the ultrasound examination only if said new step is related to the context of the ultrasound exam at the point in the protocol where it is added;
wherein the new step includes a diagnostic tool appropriate to the function of the new protocol step;
wherein a step of the exam protocol guides a user in the examination of a particular anatomical feature, wherein the new step is related to the examination of the particular anatomical feature;
wherein the new step is configured to guide the user through additional detailed analysis of the particular anatomical feature,
so as to enable the user to branch into selective, more detailed steps of the ultrasound examination and then to continue with the exam protocol to its conclusion.

2. The ultrasonic diagnostic imaging system of Claim 1, wherein the optional user selection further includes means for enabling the user to identify the new protocol step.

3. The ultrasonic diagnostic imaging system of Claim 1, wherein the diagnostic tool comprises at least one of a measurement tool and a calculation tool.

4. The ultrasonic diagnostic imaging system of Claim 3, wherein the measurement tool is one of a distance measurement, an area measurement, a volume flow measurement, or a velocity measurement.

5. The ultrasonic diagnostic imaging system of Claim 1, wherein a plurality of the steps of an exam protocol guides a user in the examination of a plurality of anatomical features, wherein the new step is related to the examination of the particular anatomical feature at the point in the protocol where the new step is added.

6. The ultrasonic diagnostic imaging system of Claim 1, further comprising means for executing an added step of a protocol.

7. The ultrasonic diagnostic imaging system of Claim 6, wherein the means for executing an added step of a protocol includes means for launching a diagnostic tool of the new protocol step in conjunction with an ultrasound image.

8. The ultrasonic diagnostic imaging system of Claim 1, further comprising means for indicating the completion of an added step of an exam protocol.

9. The ultrasonic diagnostic imaging system of Claim 1, further comprising an automatic examination report subsystem linked to the exam protocol, wherein the results of pre-programmed protocol steps and newly added protocol steps are recorded by the report subsystem.

10. The ultrasonic diagnostic imaging system of Claim 9, wherein the results of a newly added protocol step is recorded in association with a pre-programmed protocol step at the point in the protocol where the new protocol step is added.

## Patentansprüche

1. System zur diagnostischen Ultraschallbildgebung (10), das dafür ausgelegt ist, ein vorprogrammiertes Protokoll auszuführen, um einen Benutzer durch die Schritte einer Ultraschalluntersuchung zu leiten, wobei das System zur diagnostischen Ultraschallbildgebung Folgendes umfasst:
eine Anzeige (16) der Schritte eines Untersuchungsprotokolls;
eine optionale Benutzerauswahl, durch die ein Benutzer während der Ultraschalluntersuchung nur dann einen neuen Schritt zu dem Untersuchungsprotokoll hinzufügen kann, wenn sich der genannte neue Schritt auf den Kontext der Ultraschalluntersuchung an der Stelle in dem Protokoll bezieht, an der er hinzugefügt wird;
wobei der neue Schritt ein Diagnosewerkzeug umfasst, das der Funktion des neuen Protokollschritts entspricht;
wobei ein Schritt des Untersuchungsprotokolls einen Benutzer bei der Untersuchung eines bestimmten anatomischen Merkmals leitet, wobei sich der neue Schritt auf die Untersuchung des bestimmten anatomischen Merkmals bezieht;
wobei der neue Schritt konfiguriert ist, um den Benutzer durch die zusätzliche ausführliche Analyse des bestimmten anatomischen Merkmals zu leiten,
um dem Benutzer auf diese Weise zu ermöglichen, in selektive, detailliertere Schritte der Unterschalluntersuchung zu verzweigen und dann mit dem Untersuchungsprotokoll bis zu seinem Abschluss fortzufahren.

2. System zur diagnostischen Ultraschallbildgebung nach Anspruch 1, wobei die optionale Benutzerauswahl weiterhin Mittel umfasst, die es dem Benutzer ermöglichen, den neuen Protokollschritt zu kennzeichnen.

3. System zur diagnostischen Ultraschallbildgebung nach Anspruch 1, wobei das Diagnosewerkzeug mindestens entweder ein Messwerkzeug oder ein Berechnungswerkzeug umfasst.

4. System zur diagnostischen Ultraschallbildgebung nach Anspruch 3, wobei das Messwerkzeug entweder eine Entfernungsmessung, eine Flächenmessung, eine Volumenstrommessung oder eine Geschwindigkeitsmessung umfasst.

5. System zur diagnostischen Ultraschallbildgebung nach Anspruch 1, wobei eine Vielzahl der Schritte eines Untersuchungsprotokolls einen Benutzer bei der Untersuchung einer Vielzahl von anatomischen Merkmalen leitet, wobei sich der neue Schritt auf die Untersuchung des bestimmten anatomischen Merkmals an der Stelle in dem Protokoll bezieht, an der der neue Schritt hinzugefügt wird.

6. System zur diagnostischen Ultraschallbildgebung nach Anspruch 1, weiterhin mit Mitteln zum Durchführen eines hinzugefügten Schritts eines Protokolls.

7. System zur diagnostischen Ultraschallbildgebung nach Anspruch 6, wobei die Mittel zum Durchführen eines hinzugefügten Schritts eines Protokolls Mittel zum Starten eines Diagnosewerkzeugs des neuen Protokollschritts in Verbindung mit einem Ultraschallbild umfassen.

8. System zur diagnostischen Ultraschallbildgebung nach Anspruch 1, das weiterhin Mittel zum Angeben der Beendigung eines hinzugefügten Schritts eines Untersuchungsprotokolls umfasst.

9. System zur diagnostischen Ultraschallbildgebung nach Anspruch 1, das weiterhin ein automatisches Untersuchungsbericht-Subsystem umfasst, das mit dem Untersuchungsprotokoll verknüpft ist, wobei die Ergebnisse der vorprogrammierten Protokollschritte und der neu hinzugefügten Protokollschritte durch das Bericht-Subsystem aufgezeichnet werden.

10. System zur diagnostischen Ultraschallbildgebung nach Anspruch 9, wobei die Ergebnisse eines neu zugefügten Protokollschritts in Zusammenhang mit einem vorprogrammierten Protokollschritt an der Stelle in dem Protokoll, an der der neue Protokollschritt hinzugefügt wurde, aufgezeichnet werden.

## Revendications

1. Système d'imagerie de diagnostic ultrasonore (10) apte à exécuter un protocole préprogrammé pour guider un utilisateur à travers les étapes d'un examen ultrasonore, comprenant :
un affichage (16) des étapes d'un protocole d'examen ;
une sélection d'utilisateur facultative par laquelle un utilisateur peut ajouter une nouvelle étape au protocole d'examen au cours de l'examen ultrasonore uniquement si ladite nouvelle étape est relative au contexte de l'examen ultrasonore au point dans le protocole où elle est ajoutée ;
dans lequel la nouvelle étape comprend un outil de diagnostic approprié à la fonction de la nouvelle étape de protocole ;
dans lequel une étape du protocole d'examen guide un utilisateur dans l'examen d'une caractéristique anatomique particulière, dans lequel la nouvelle étape est relative à l'examen de la caractéristique anatomique particulière ;
dans lequel la nouvelle étape est configurée pour guider l'utilisateur à travers une analyse détaillée supplémentaire de la caractéristique anatomique particulière ;
de manière à permettre à l'utilisateur d'accéder à des étapes sélectives plus détaillées de l'examen ultrasonore puis de poursuivre le protocole d'examen jusqu'à sa conclusion.

2. Système d'imagerie de diagnostic ultrasonore selon la revendication 1, dans lequel la sélection d'utilisateur facultative comprend en outre des moyens pour permettre à l'utilisateur d'identifier la nouvelle étape de protocole.

3. Système d'imagerie de diagnostic ultrasonore selon la revendication 1, dans lequel l'outil de diagnostic comprend au moins l'un d'un outil de mesure et d'un outil de calcul.

4. Système d'imagerie de diagnostic ultrasonore selon la revendication 3, dans lequel l'outil de mesure effectue l'une d'une mesure de distance, d'une mesure de surface, d'une mesure de débit volumétrique ou d'une mesure de vélocité.

5. Système d'imagerie de diagnostic ultrasonore selon la revendication 1, dans lequel une pluralité des étapes d'un protocole d'examen guident un utilisateur dans l'examen d'une pluralité de caractéristiques anatomiques, dans lequel la nouvelle étape est relative à l'examen de la caractéristique anatomique particulière au point dans le protocole où la nouvelle étape est ajoutée.

6. Système d'imagerie de diagnostic ultrasonore selon la revendication 1, comprenant en outre des moyens pour exécuter une étape ajoutée d'un protocole.

7. Système d'imagerie de diagnostic ultrasonore selon la revendication 6, dans lequel les moyens pour exécuter une étape ajoutée d'un protocole comprennent des moyens pour lancer un outil de diagnostic de la nouvelle étape de protocole en relation avec une image ultrasonore.

8. Système d'imagerie de diagnostic ultrasonore selon la revendication 1, comprenant en outre des moyens pour indiquer l'achèvement d'une étape ajoutée d'un protocole d'examen.

9. Système d'imagerie de diagnostic ultrasonore selon la revendication 1, comprenant en outre un sous-système de rapport d'examen automatique lié au protocole d'examen, dans lequel les résultats des étapes de protocole préprogrammées et des nouvelles étapes de protocole ajoutées sont enregistrés par le sous-système de rapport.

10. Système d'imagerie de diagnostic ultrasonore selon la revendication 9, dans lequel les résultats d'une nouvelle étape de protocole ajoutée sont enregistrés en association avec une étape de protocole préprogrammée au point dans le protocole où la nouvelle étape de protocole est ajoutée.
